(19) Europäisches Patentamt
European Patent Office
Office européen des brevets

(11) Publication number: **0 159 513**
**A1**

(12) **EUROPEAN PATENT APPLICATION**

(21) Application number: **85102796.1**

(22) Date of filing: **12.03.85**

(51) Int. Cl.⁴: **C 12 Q 1/00**, G 01 N 33/84
// C12Q1/26

(30) Priority: **22.03.84 IT 4790784**

(43) Date of publication of application: **30.10.85**
**Bulletin 85/44**

(84) Designated Contracting States: **AT BE CH DE FR GB LI NL SE**

(71) Applicant: **Miles Italiana SpA, Via FL Miles 10, I-20040 Cavenago Brianza (Mi) (IT)**

(72) Inventor: **Fossati, Piero, Via M. Buonarroti 36, I-20035 Lissone Milano (IT)**

(74) Representative: **Adrian, Albert, Dr. et al, c/o BAYER AG Konzernverwaltung RP Patentabteilung, D-5090 Leverkusen 1, Bayerwerk (DE)**

(54) **Enzymatic inorganic phosphate assay.**

(57) A test composition, device and method for the enzymatic determination of inorganic phosphates. The composition comprises inosine, xanthine oxidase, purine-nucleoside phosphorylase, and a tetrazolium salt capable of generating a detectable response. In a preferred embodiment the composition additionally includes an electron mediator and a buffer capable of providing a pH in the range of from about 5.0 to 12.0. After contacting the fluid sample with the assay composition, the concentration of inorganic phosphate can be determined instrumentally or visually if an appropriate color chart is supplied.

EP 0 159 513 A1

# ENZYMATIC INORGANIC PHOSPHATE ASSAY

## FIELD OF THE INVENTION

The present invention relates generally to the field of test compositions and, more particularly, to test compositions useful for determination of inorganic phosphates in fluids, such as urine or blood (serum or plasma).

## BACKGROUND OF THE INVENTION

An assay for inorganic phosphates, $HPO_4^{-2}$ and $H_2PO_4^-$, is especially useful as a diagnostic tool. For example, serum inorganic phosphate concentration is elevated in healing bone fractures, hypoparathyroidism, Paget's disease, acromegaly, dehydration; and is decreased in vitamin D deficiency, malabsorption syndromes, hyperinsulism, asteomalacia, hyperparathyroidism and the like.

Prior art methods for determining inorganic phosphates (Pi) in biological fluids can be grouped into two categories: chemical and enzymatic methods.

All the chemical methods require strongly acidic conditions. The resulting hydrolysis of unstable organic phosphates present in biological fluids causes erroneously high values for inorganic phosphate.

Several enzymatic methods for the determination of inorganic phosphate have been proposed. Schulz, D.W. *et al., 19, Anal. Bioch.*, 300 (1967), proposed an enzymatic method for the determination of inorganic phosphate based on the use of phosphorylase,

phosphoglucomutase and glucose-6-phosphate dehydrogenase. The reduced form of nicotine adenine dinucleotide (NADH) is generated. The NADH formed is monitored spectrophotometrically in the ultraviolet region and is directly proportional to the concentration of inorganic phosphate in the sample. Scopes, R.K., *49, Anal. Biochem.,* 88 (1972) proposed the use of an alternative enzymatic route in which phosphate assay is based on the use of glyceraldehyde-3-phosphate dehydrogenase. The resulting assay also required monitoring of NADH in the ultraviolet region of the spectrum.

A third enzymatic pathway for the assay of inorganic phosphate was proposed by Misaka et al., in U.S. Patent No. 4,246,342 based on the use of pyruvate oxidase. The hydrogen peroxide generated was monitored in the visual range with a Trinder type indicator system, 4-aminophenazone and a substituted aniline.

W. I. Hwang, *et al., 55, Anal. Biochem.,* 379 (1973), proposed another enzymatic method for the determination of inorganic phosphate:

$$\text{Inosine} + \text{Pi} \xrightarrow{\text{Purine-nucleoside phosphorilase}} \text{Hypoxanthine} + \text{Ribose-1-phosphate}$$

$$\text{Hypoxanthine} + 2H_2O + 2O_2 \xrightarrow[\text{(EC 1.2.3.2)}]{\text{Xanthine oxidase}} \text{Uric acid} + 2H_2O_2$$

wherein the uric acid produced is monitored in the ultraviolet range of the spectrum at 290 nanometers (nm). This method, however, was not applied to biological samples, where the presence of large amounts of endogenous uric acid would require a sample blank.

Klebe R. J., *et al.*, *19*, *Biochem. Genetics* 921 (1981) utilized the Hwang reaction sequence to visualize gel electrophoresis bands of phosphate generating enzymes, using Nitroblue Tetrazolium as a chromogen. The method was used as a qualitative means of detecting isozymes which liberate inorganic phosphate.

Machida, Y. *et al.*, *46*, *Agri. Biol. Chem.*, 807 (1982), utilized the Hwang reaction sequence for the assay of inorganic phosphate. When a particular xanthine oxidase obtained from *Enterobacter cloacae* was used in the assay, the concentration of Pi could be measured in the visible range by following the production of hydrogen peroxide with a Trinder indicator system, 4-aminoantipyrine and phenol. Machida states that a quantative assay in the visible region could not be accomplished with milk xanthine oxidase. Bovine milk xanthine oxidase is the only commercially available xanthine oxidase source.

None of these enzymatic methods provides a sensitive, accurate convenient assay for inorganic phosphate from commercially available materials.

## *SUMMARY OF THE INVENTION*

The present invention provides a sensitive, accurate, reproducible assay for inorganic phosphate. Since the assay utilizes readily available commercial materials and is monitored in the visual range of the spectrum, it is convenient for routine clinical application.

A test composition, device and method are disclosed which enable the enzymatic assay of inorganic phosphates in a fluid sample including body fluids, such as urine and blood (serum or plasma). The

composition comprises inosine, purine-nucleoside phosphorylase, xanthine oxidase and a tetrazolium salt.

A fuller understanding of the invention will be attained by referring to the following description and claims.

*DESCRIPTION OF THE PREFERRED EMBODIMENTS*

The assay composition includes ionosine, purine-nucleoside phosphorylase, xanthine oxidase and a tetrazolium salt.

Additionally, a buffering substance capable of providing a pH in the range of from about 5 to 12, must be present. The buffer can be incorporated into the assay composition or added to the sample prior to assaying for phosphate content. Preferably, the assay composition also includes an electron mediator. Other components, such as stabilizers, surfactants or dispersants can be added without departing from the scope of the invention.

The concentration of formazan formed is proportional to the phosphate concentration at any given time, therefore either an end-point method or a rate assay method can be used.

The measurement of inorganic phosphate is accomplished according to the following sequence of reactions:

1) Inosine + Pi —Purine-Nucleoside Phosphorylase (EC 2.4.2.1)→ Ribose-1-phosphate + Hypoxanthine

2) Hypoxanthine + tetrazolium salt —xanthine oxidase (EC 1.2.3.2)→ xanthine + formazan

3) Xanthine + tetrazolium salt —xanthine oxidase (EC 1.2.3.2)→ uric acid + formazan

All the assay components are commercially available. A particularly preferred xanthine oxidase is that obtained from bovine milk, although any xanthine oxidase can be used.

The tetrazolium salt can be either 2,3,5-aromatic substituted derivatives of 1,2,3,4-tetrazole, or ditetrazolium compounds in which two tetrazolium rings are linked by an aromatic grouping. Examples of useful tetrazolium salts are 3-(4',5'-dimethyl-2-thiazolyl)-2,4-diphenyl-2H-tetrazolium bromide (MTT or Thiazolyl Blue), 2-(p-iodophenyl)-3-(p-nitrophenyl)-5-phenyl-2H-tetrazolium chloride (INT); 3,3'-(3,3'-dimethyoxy-4,4'-diphenylene)-bis-(2-p-nitrophenyl-5-phenyl)-2H-tetrazolium chloride (Nitroblue tetrazolium or NBT); and 3,3'-(dimethoxy-4,4'-diphenylene)-bis-(2,5-p-nitrophenyl)-2H-tetrazolium chloride (Tetranitroblue tetrazolium or TNBT). The tetrazolium salt, 3-(4',5'-dimethyl-2-thiazolyl)-2,4-diphenyl-2H-tetrazolium bromide (MTT or Thiazolyl Blue) is particularly preferred.

Formazan color formation by the tetrazolium salt indicator can be accelerated by the addition of an electron mediator. Phenazine salts, such as phenazine methosulfate and 1-methoxy phenazine methosulfate, and other compounds, such as 8-dimethyl-amino-2,3-benzophenoxazine (Meldola Blue), 7-amino-3-imino-3H-phenothiazine (Thionine) or tetramethylamidophenthiazine chloride (methylene Blue) can be used. A preferred electron mediator is 1-methoxy-phenazine methosulfate. Suitable buffers for incorporation into the assay composition or addition into the sample before assay include Tris (hydroxymethyl)aminomethane N-2-hydroxyethylpiperazine-N'-2-ethanesulfonic acid 3-(N-morpholino) propanesulfonic acid, N,N'-bis (2-hydroxyethyl)-2-aminoethanesulfonic acid, N-tris (hydroxymethyl) methyl-2-aminoethanesulfonic

acid, N-2-hydroxyethylpiperazine propanesulfonic acid and diethanolamine. All the above are capable of providing a pH in the range of from about 5 to 12. In a preferred embodiment the pH is within the range of 6.0 to 11.0.

Workable and preferred concentration ranges are shown below:

| | Workable | Preferred |
|---|---|---|
| Inosine | 0.01-10 mM | 0.1-2 mM |
| Purine-nucleoside Phosphorylase | 1-1000 I.U./L | 10-300 I.U./L |
| Xanthine oxidase | 0.1-1500 I.U./L | 1-100 I.U./L |
| Tetrazolium salt | 0.05-30 mM | 0.1-5 mM |
| Electron mediator | 0-1000 μM | 1-200 μM |
| Buffer pH | 5.0-12.0 | 6.0-11.0 |
| Buffer molarity | 10-1000 mM | 30-300 mM |
| Surfactants | 0-10% (w/v) | 0.1-3% (w/v) |

Units: mM (millimolar), μM (micromolar), %w/v (percent weight per volume), I.U. is an International Unit of enzyme activity. One I.U. is the enzyme activity required to catalyze the conversion of one micromole of substrate per minute at specified conditions of pH and temperature. For purine-nucleoside phosphorylase, the I.U. indicated above is measured for the substrate inosine at pH 7.4 and 25°C. For xanthine oxidase, the I.U. above is measured for the substrate hypoxanthine at pH 7.6 and 25°C. One International Unit per liter (I.U./L) is one International Unit of enzyme activity per liter (L) of stock solution.

The composition can be used in aqueous solution form prepared with solvents, such as water, physiological solution, or in mixtures of water and organic solvents, such as methanol. The reagents can be provided in a "kit" format, e.g., a series of bottles containing one or more of the above components, either in solution or as a premixed lyophilized powder or as a tablet which can be reconstituted for use as a single working solution. The kit can contain optionally a suitable phosphate standard.

Alternatively, the composition can be incorporated into a carrier matrix to provide a dry test device. In either case the assay is performed by contacting a sample containing inorganic phosphate with the assay composition and measuring the color formed by the formazan dye. The color formation is usually measured instrumentally in the visible range, generally between 500 and 600 nanometers, with a colorimeter or visible photometer. Since the color developed at any given time is proportional to the phosphate concentration, either an end-point method or a rate assay method can be used. Alternatively, the color formation can be determined with visual reading alone if an appropriate color chart is supplied, resulting in a semiquantitative assay.

The term "carrier matrix", as used herein, refers to any means suitable for containing specified amounts of the test composition. Many such materials are known. See, for example, U.S. Patent Nos. 3,846,247; 3,522,928; 4,046,513, 4,046,514; French Patent No. 2,170,397 and British Patent Nos. 1,369,139 and 1,340,623 among others. It is, therefore, to be appreciated that in producing a test device of the invention, all such carrier matrix concepts can be employed, as can others. The matrix can include a

system which physically entraps any or all of these ingredients, such as polymeric microcapsules which rupture upon contact with an aqueous solution. The presently preferred method of preparing a dry test device, however, is to impregnate a porous carrier matrix, e.g., filter paper, with the composition, followed by affixing of the impregnated matrix to a support member. The impregnation can be accomplished merely by dipping one piece of filter paper into a solution of the composition. Drying can be accomplished by any means which will not deleteriously affect the impregnated composition, usually by means of an air oven. The dried paper can thereafter be cut and mounted on one end of a support member, for example, a rigid or semi-rigid polystyrene film strip. Mounting of the paper on the strip can be accomplished through use of a double-faced adhesive tape, such as that commercially available from the 3M Co. as DOUBLE STICK®. The test device is conveniently used by momentarily dipping it in a test sample or by otherwise introducing a test sample into the carrier matrix, whereby a detectable change (e.g., color) results when inorganic phosphate is present.

The following, Examples I and II describe an assay for inorganic phosphate performed using the test composition of the present invention in solution form. Example III illustrates a procedure for preparing a test device. While the examples serve to illustrate the invention, they are not to be interpreted as limiting its scope, which is defined solely by the claims appended hereto. One skilled in the art will be able to make such variations, substitutions and changes in the components of the composition and ingredients and reaction parameters as may seem desirable.

*EXAMPLE I*

Test Composition, Solution Form

The test composition was prepared in aqueous solution according to the formulation below. The chemicals and enzymes used are commercially available. Since the assay is directed toward a determination of phosphate, all preparations were essentially phosphate free.

Test Composition

| | |
|---|---|
| inosine | 0.45 mM |
| purine-nucleoside phosphorylase | 90 I.U./L |
| xanthine oxidase | 9 I.U./L |
| 3-(4',5'-dimethyl-2-thiazolyl)-2-4-diphenyl-2H-tetrazolium bromide | 0.6 mM |
| 1-methoxy-phenazine methosulfate | 30 μM |
| Tris(hydroxymethyl)aminomethane, pH 7.50 | 150 mM |
| Surfactant (Triton® X-100) | 0.3% w/v |

A series of phosphate standards was prepared. A 2.0 milliliters (ml) portion of the above test composition was added to a 0.02 ml aliquot of each of the phosphate standards and absorbance measurements taken at 578 nanometers (1 centimeter pathlength) after 15 minutes. Absorbance of a reagent blank was subtracted from the test absorbance to give the true absorbance value. The values given were plotted (absorbance versus phosphate concentration in milligrams per deciliter) to provide a standard curve. Absorbance of the phosphate standards was linear with increasing phosphate concentration (see Table I).

TABLE I

| phosphate (mg/dl) | absorbance |
|---|---|
| 2.5 | 0.263 |
| 5.0 | 0.526 |
| 7.5 | 0.785 |
| 10.0 | 1.050 |
| 15.5 | 1.579 |

Body fluid samples were tested in a similar manner. Sample phosphate concentration was determined by comparing the true sample absorbance values to the standard curve.

*EXAMPLE II*

Percent Recovery of Inorganic Phosphate Added to a Serum Pool

When known amounts of phosphate were added to sera, or pools of sera, previously assayed for phosphate, the percent recovery (total phosphate found minus phosphate originally found expressed as a percent of phosphate added) averaged 100.4% (see Table II).

TABLE II

Percent Recovery of Inorganic Phosphate Added to a Serum Pool

| Phosphate added (mg/dl) | Percent recovery |
|---|---|
| 1.25 | 99.5 |
| 2.50 | 100.0 |
| 5.00 | 101.0 |
| 8.00 | 99.0 |
| 11.00 | 102.0 |
| 15.00 | 101.0 |
| average: | 100.4 |

This recovery demonstrates that the composition of the present invention is capable of quantitative measurement of phosphate in solution.

*EXAMPLE III*

Preparation of a Test Device

A test device for the enzymatic determination of inorganic phosphates is prepared by incorporating the test composition of Example I into a carrier matrix. For example, Whatman® No. 17 filter paper can be immersed in a solution containing the test composition and dried at 60°C in an air oven. A 2.5 x 2.5 centimeter square of the dried impregnated paper is affixed to a polystyrene strip with double-faced adhesive tape to form a test device.

The device is conveniently used by momentarily dipping it into a fluid sample or by otherwise introducing the sample into the carrier matrix.

A detectable color change results when inorganic phosphate is present. Since the color developed depends on inorganic phosphate concentration, a semiquantitative assay is possible when an appropriate color chart is supplied. A quantitative assay is possible when an appropriate reflectance reading instrument is used.

Other formats such as a tablet or reagent matrix can be used to prepare the test device.

Obviously, many other modifications and variations of the invention as set forth may be made without departing from the spirit or scope thereof.

WHAT IS CLAIMED IS:

1. A composition for the enzymatic determination of inorganic phosphates in a fluid sample, characterized in that it comprises:

inosine, purine-nucleoside phosphorylase, xanthine oxidase and a tetrazolium salt.

2. The composition of claim 1, characterized in that the xanthine oxidase is obtained from bovine milk.

3. The composition of any of claims 1 or 2, characterized in that it additionally comprises an electron mediator.

4. The composition of any of claims 1 to 3, characterized in that it additionally comprises a buffering substance capable of providing a pH in the range of from about 5 to 12.0.

5. The composition for the enzymatic determination of inorganic phosphates according to claim 1, characterized in that the xanthine oxidase is obtained from bovine milk, and which additionally contains an electron mediator and a buffering substance capable of providing a pH in the range of from about 6.0 to 11.0.

6. A test device for the enzymatic determination of inorganic phosphates, characterized in that it comprises a carrier matrix incorporated with a composition according to any of claims 1 to 4.

7. A test device for the enzymatic determination of inorganic phosphates according to claim 6, characterized in that the xanthine oxidase is obtained from bovine

milk and which carrier matrix is additionally incorporated with an electron mediator and a buffering substance capable of providing a pH in the range of from about 6.0 to 11.0.

8. A process for the enzymatic determination of inorganic phosphates in a fluid sample, characterized in that it comprises

a) contacting the sample with a buffer capable of providing a pH in the range of from about 5.0 to about 12.0; and

b) a composition according to any of claims 1 to 3,

or a test device comprising a carrier matrix incorporated with this composition.

9. A process for the enzymatic determination of inorganic phosphates in a fluid sample, characterized in that it comprises contacting the sample with

a) a composition according to any of claims 1 to 4, or with

b) a test device comprising a carrier matrix incorporated with the composition.

10. A method for preparation of a test device for enzymatic determination of inorganic phosphate characterized in that a composition comprising inosine, purine-nucleosid phosphorglase, xanthin oxidase, a tetrasolium salt, an electron medicator and a buffering substance capable of providing a pH in the range from about 5 to 12 is incorporated in a carrier matrix.

European Patent Office

# EUROPEAN SEARCH REPORT

0159513 Application number

EP 85 10 2796

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | CHEMICAL ABSTRACTS, vol. 96, no. 23, 7th June 1982, page 342, no. 195993p, Columbus, Ohio, US; Y. MACHIDA et al.: "Utilization of bacterial xanthine oxidase for inorganic phosphorus determination" & AGRIC. BIOL. CHEM. 1982, 46(3), 807-808 * Abstract * --- | 1-10 | C 12 Q 1/00<br>G 01 N 33/84 //<br>C 12 Q 1/26 |
| Y | CHEMICAL ABSTRACTS, vol. 82, no. 5, 3rd February 1975, page 253, no. 28147v, Columbus, Ohio, US; R. FRIED et al.: "Hypoxanthine and xanthine. Color test" & METHODEN ENZYM. ANAL., 3. NEUBEARBEITETE ERWEITERTE AUFL. 1974, 2, 1992-1998 * Abstract * --- | 1-10 | TECHNICAL FIELDS SEARCHED (Int. Cl.4) |
| D,Y | CHEMICAL ABSTRACTS, vol. 79, no. 19, 12th November 1973, page 139, no. 112942w, Columbus, Ohio, US; W.I. HWANG et al.: "New enzymic method for the determination of inorganic phosphate and its application to the nucleoside diphosphatase assay" & ANAL. BIOCHEM. 1973, 55(2), 379-387 * Abstract * --- -/- | 1-10 | C 12 Q<br>G 01 N |

The present search report has been drawn up for all claims

| Place of search | Date of completion of the search | Examiner |
|---|---|---|
| THE HAGUE | 01-07-1985 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

0159513 Application number

EP 85 10 2796

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-4 046 514 (K. ELKHART et al.) * Whole document * | 6-10 | |
| D,Y | CHEMICAL ABSTRACTS, vol. 95, no. 25, 21st December 1981, page 209, no. 216838p, Columbus, Ohio, US; R.J. KLEBE et al.: "Visualization of isozymes which generate inorganic phosphate" & BIOCHEM. GENET. 1981, 19(9-10), 921-927 * Abstract * | 1-10 | |
| Y | US-A-4 246 341 (J. ZIKAKIS) * Abstract; claim 1 * | 1-10 | |
| Y | EP-A-0 016 845 (KYOWA HAKKO KOGYO CO., LTD.) * Examples4,5; claims 1-9 * | 1-10 | TECHNICAL FIELDS SEARCHED (Int. Cl.4) |

The present search report has been drawn up for all claims

| Place of search | Date of completion of the search | Examiner |
|---|---|---|
| THE HAGUE | 01-07-1985 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document